# EUROPEAN PATENT APPLICATION

(11) **EP 4 760 129 A1**
(43) Date of publication of application: **17.06.2026**
(21) Application number: 24218762.3
(22) Date of filing: 10.12.2024
(51) Int. Cl.: F16K 1/42, F16K 25/00, F16K 27/02, B01J 19/02

(54) **A PRESSURE CONTROL VALVE COMPRISING A VALVE SEAT**

(71) Applicant: Yara International ASA, 0277 Oslo (NO)
(72) Inventor: Porro, Lino Giovanni, 1040 Etterbeek (BE); Paci, Giulio, 05035 Narni (IT); Caporusso, Carlotta, 40061 Minerbio (BO) (IT); Gobbi, Stefano, 44122 Ferrara (IT)
(74) Representative: De Clercq & Partners

(57) **Abstract**

The present disclosure provides a pressure control valve (1), in particular for use in a urea-producing plant. The present disclosure also provides a urea-producing plant comprising one or more pressure control valves (1), and the use of a pressure control valve (1) in a urea-producing plant. The pressure control valve (1) comprises a first body part (2) and a second body part (3) fluidically connected to each other through a valve seat (8), wherein the first body part (2) comprises an inlet (4) and a flow-regulating component, the movement of which regulates the volume flow rate of fluid flowing from the first body part (2) to the second body part (3); the second body part (3) comprises an outlet (5); the first body part (2) and the second body part (3) are made from the same first material; wherein the valve seat (8) has a cylindrical shape and circular walls and comprises an inner region (10) and an outer region (9), and wherein the outer region (9) is made from a second material, the inner region (10) is made from a third material, and the second material has a lower hardness than the third material.

## Description

### Field of the disclosure

The present disclosure is related to the field of chemical engineering.

### Background information

Industrial plants, such as urea-producing plants, comprise devices that operate with fluids with varying pressures. In order to operate these devices and transfer fluids between these, pipes and pressure control valves are required. The valves should be able to tolerate high pressures, high temperatures and corrosive compositions.

There are several types of designs for pressure control valves. These designs have different advantages and disadvantages, so each valve is chosen carefully for its purpose.

Urea plants have different sections operating at various pressure (up to over 200 bar) and temperatures (over 140 °C, and up to 210 °C). Many streams in a urea plant comprise ammonium carbamate, which is a highly corrosive material. This requires the use of devices and valves made of particular materials, such as austenitic, duplex, and super duplex stainless steels.

There is a need to develop new valve designs that are more resistant to the operating conditions of a urea plant and easier to maintain.

### Summary of the disclosure

It was found that a pressure control valve comprising a valve seat made of at least two regions made of a different material presented several advantages. The pressure control valve seat comprises an inner region made of a hard, highly corrosion-resistant material, to ensure reliability and elevated lifetime of the valve, and an outer region made of a softer material that ensures a tight seal between the two body parts of the valve, as well as high resistance to corrosion.

In a first aspect, the present disclosure provides a pressure control valve comprising a first body part and a second body part fluidically connected to each other through a valve seat, wherein:
- the first body part comprises a pressure control valve fluid inlet and a flow-regulating component, the movement of which regulates the volume flow rate of fluid flowing from the first body part to the second body part;
- the second body part comprises a pressure control valve fluid outlet;
- the first body part and the second body part are made from the same first material; characterized in that
   the valve seat has a cylindrical shape and circular walls and comprises an inner region and an outer region, wherein the outer region is made from a second material, the inner region is made from a third material, and the second material has a lower hardness than the third material.

In another aspect, the present disclosure provides a urea-producing plant comprising one or more pressure control valves according to the first aspect.

In another aspect, the present disclosure provides the use of a pressure control valve according to the first aspect in a urea-producing plant.

### Brief description of the figures

The following description of the figure of a specific embodiment of a system according to the present disclosure is only given by way of example and is not intended to limit the present explanation, its application or use. In the figure, identical reference numerals refer to the same or similar parts and features.

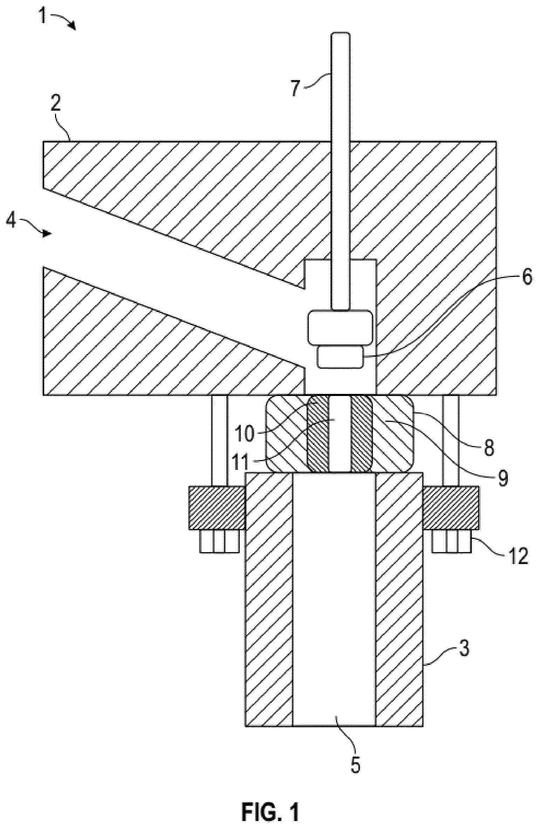

Figure 1 shows an embodiment of a pressure control valve according to the first aspect of the present disclosure.

### Detailed description of the disclosure

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

All references cited in this description are hereby deemed to be incorporated in their entirety by way of reference.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20 % or less, in particular +/-10 % or less, more in particular +/-5 % or less, even more in particular +/-1 % or less, and still more in particular +/-0.1 % or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

In a first aspect, the present disclosure provides a pressure control valve comprising a first body part and a second body part fluidically connected to each other through a valve seat, wherein:
- the first body part comprises a pressure control valve fluid inlet and a flow-regulating component, the movement of which regulates the volume flow rate of fluid flowing from the first body part to the second body part;
- the second body part comprises a pressure control valve fluid outlet;
- the first body part and the second body part are made from the same first material; characterized in that
the valve seat has a cylindrical shape and circular walls and comprises an inner region and an outer region, wherein the outer region is made from a second material, the inner region is made from a third material, and the second material has a lower hardness than the third material.

The pressure control valve is configured to receive a stream of a fluid, which may comprise at least one gas component and/or at least one liquid component, at a certain pressure via its pressure control valve fluid inlet comprised in the first body part. The fluid flows towards the second body part via the chamber of the valve seat and exits the valve via the pressure control valve fluid outlet.

The flow-regulating component is a component, which can be moved between a closed position and a fully open position, within the first body part to regulate the flow of fluid in the valve.

In the closed position, the flow-regulating component is in contact with the valve seat and no fluid can reach the second body part and the fluid outlet, effectively closing the valve.

The body of the valve comprises two body parts, the two body parts being hollow to allow the fluid for flowing from the pressure control valve fluid inlet of the valve to the pressure control valve fluid outlet. The first body part comprises the pressure control valve fluid inlet, and the second body part comprises the pressure control valve fluid outlet.

The first and second body parts are made of the same first material. The valve is configured to be used in a urea-producing plant, in particular in a high-pressure section of a urea-producing plant. This means that the valve will be in contact with liquid streams comprising ammonium carbamate at high temperature (at least 80 °C, and up to 210 °C) and high pressure (above 1.0 MPa), so the body parts of the valve need to be selected in a material that is resistant to corrosion under these conditions. Suitable materials are known to the person skilled in the art.

A critical part of a valve comprising two body parts is the junction of these body parts. This junction is a common source of leaks which cause the plant to stop. The first and second body parts are joined with each other, for example with one or more fasteners, such as screws. In addition, a valve seat is located between the first and second body and in contact with the first and second body parts. The valve seat is configured to act as a gasket between the first body part and the second body part and to guarantee a proper seal to avoid leaks in the production plant.

The valve seat has a cylindrical shape and circular walls and comprises a chamber, which is a through-hole for the fluid to flow from the first body part to the second body part.

In order to obtain the best possible joint between the first and second body part, said otherwise, to obtain the device with the lowest leak risk between the first and second body part, it has been found that the valve seat should have two regions of different hardness.

When the valve is at least partially open, the inner region of the valve seat is in contact with a fluid that may contain corrosive components, such as ammonium carbamate, with a high velocity. The high velocity increases the corrosive effect of the solution and can lead to higher corrosion rates. It is therefore important to use a material with a high corrosion resistance. However, it has been observed that materials with higher corrosion resistance than other materials have also often a higher hardness.

However, a material with a high hardness is less likely to change shape under pressure. Since the valve seat is configured to act as a gasket between the first and second body parts, a high hardness can lead to a less tight joint between the first and second body parts and the valve seat, increasing the risk of leakage.

The outer region of the valve seat may also be in contact with the fluid flowing through the valve, but the velocity on the outer region is less than on the inner region, and the corrosion rate is lower, so a different material with a slightly lower corrosion resistance may be used without compromising on the expected lifetime of the valve.

It is therefore an advantage for the valve seat to be made of two materials: a hard, highly corrosion resistance material in its inner region, and a softer material in its outer region to ensure a good seal with the first and second body parts.

In some embodiments, the flow-regulating component comprises a stem.

The flow-regulating component may comprise a stem connected to the disc. The stem is usually straight and may extend beyond the body of the valve.

The stem may be attached to a controlling device, such as a handwheel or an automatic actuator (pneumatic or electrical), which can be manipulated by a user to modify the position of the disc inside the valve.

In some embodiments, the flow-regulating component comprises a disc. The flow-regulating component may comprise a disc, which is in contact with the valve set when the valve is closed. As used herein, the term "disc" refers to the component that comes into contact with the valve seat and is widely used in the field. The term "disc" does not provide any indication on the shape of that component.

In some embodiments, the stem of the flow-regulating component is made of the third material.

In some embodiments, the disc is made of the third material.

In some embodiments, the stem of the flow-regulating component is made of a super duplex stainless steel.

In some embodiments, the disc is made of a super duplex stainless steel.

In some embodiments, the disc is made of UNS S32906 or UNS S32550. A UNS number is short for the Unified Numbering System for Metals and Alloys. It is a classification system for metal alloys. Each UNS number refer to an alloy composition comprising different metals, each metal having a specific content range. UNS S32906, UNS32808 (sold under the trademark DP28W^{™}) and UNS S32550 are super duplex stainless steels.

In some embodiments, the hardness of the second material is lower than that of the first material and the third material. It was found that it may be more practical for the second material present in the outer region of the valve seat is softer, i.e., has a lower hardness than the first and second body parts. The difference in hardness allows the softer material, i.e. the material with the lowest hardness, to deform enough to obtain a tight join between the valve seat and the first body part, and between the valve seat and the second body part.

In some embodiments, the first material and the third material are identical.

In some embodiments, the first material is an austenitic stainless steel, a duplex stainless steel or a super-duplex stainless steel. Austenitic stainless steel is a class of stainless steel with an austenitic crystalline structure. Duplex stainless steel is a class of stainless steel with a ferritic crystalline structure including islands with austenitic structures. Various austenitic stainless steels are known to be suitable for use in urea-producing plants, such as UNS S31603, and UNS S31050. UNS 31603 is also known as ASTM 316L, 316L MOD, and 316L Urea Grade. UNS S31050 is also known as 25.22.2, corresponding to the respective content in chromium, nickel, and molybdenum. Suitable duplex stainless steels include UNS S32305, and UNS S31260.

In some embodiments, the third material is a super duplex stainless steel. Super duplex stainless steel is a sub-category of duplex stainless steel. The inner region of the valve seat, in particular the chamber comprised therein, is in contact with the fluid flowing through the valve, unless the valve is completely closed. So, the inner region should be in a material highly resistant to ammonium carbamate corrosion. In addition, the velocity of fluid is greater in the chamber than in the rest of the valve, creating a higher risk of erosion. Super duplex stainless steel compositions have been developed with the focus on corrosion/erosion resistance, so these materials are particularly suitable to be used in the inner region of a valve seat. Examples of super duplex stainless steel suitable for urea-producing plants include UNS S32906, sold under the brands Safurex^{®} and Uremium^{®}, UNS S32550, sold under the brands Ferralium255 and HVD1^{™}.

In some embodiments, the second material is an austenitic stainless steel. The main role of the outer region is to ensure a good seal between the first body part and the second body part, such that the fluid entering the valve does not leak out of the valve at the junction between the first body part and the second body part. The valve is to be used in a urea-producing plant, so it should be made of a material that has a reasonably good resistance to carbamate corrosion. It has been found that if the first and second body parts of the valve are made of a duplex or super duplex stainless steel, it was an advantage that the outer region of the valve seat is made of an austenitic stainless steel. Some austenitic stainless steels are known to have a good resistance towards carbamate corrosion, such as UNS S31603 and UNS S31050. These materials may also have a lower hardness than super duplex stainless steels. When the two body parts are joined with each other, the softer outer region of the valve seat is able to deform slightly and ensures a tight join between the two body parts, plus resisting to carbamate corrosion. If the first and second body parts of the valve are made of an austenitic stainless steel, an outer region of the valve seat in austenitic stainless steel can also provide a good seal between the valve seat and the body parts.

In some embodiments, the first material and second material belong to the same class of material, for example austenitic stainless steels. It may be possible for the first and second body parts, and the outer region of the valve seat to be made in materials from the same class, in particular austenitic stainless steels. Several austenitic stainless steels have a hardness that provides enough deformation to ensure a good seal between the first and second body parts and the valve seat.

In some embodiment, the flow-regulating component is selected from the group consisting of a parabolic-type disc, a needle-type disc, a ball-type disc, and a plug-type disc. The valve comprises a flow-regulating component, which can be moved inside the valve to increase or decrease the flow of fluid going through the valve seat. The flow-regulating component may have various geometries and designs, which are known in the art.

In another aspect, the present disclosure provides a urea-producing plant comprising one or more pressure control valves according to the first aspect.

A urea producing plant comprises several sections, such as a synthesis section, a recovery or decomposition section, a concentration section, and a finishing section.

The synthesis section is configured to produce an aqueous urea solution comprising urea, water, and ammonium carbamate, from ammonia and carbon dioxide.

The recovery section, also called decomposition section, is connected to the synthesis section and configured to receive an aqueous urea solution from the synthesis section and transform the aqueous urea solution into a pure urea solution comprising urea, water, and less than 5.0 weight% of impurities.

The evaporation section is connected to the decomposition section and configured to remove water from the pure urea solution comprising urea, water, and less than 5.0 weight% of impurities, from the recovery section to obtain a urea melt comprising urea and less than 5.0 weight% of water.

The finishing section is configured to receive a urea melt and produce a urea-containing product, the urea-containing product may be solid or liquid.

In some embodiments, the urea-producing plant comprises a condensation section connected to the decomposition section and/or the evaporation section. The condensation section may be configured to transform gaseous streams comprising ammonia, carbon dioxide and water, from the decomposition section and/or the evaporation section, into an aqueous solution comprising ammonium salts. The condensation section may also be connected to the synthesis section and configured to send an aqueous solution comprising ammonium salts to the synthesis section. The condensation section may comprise at least one carbamate condenser configured to produce low-pressure steam from the condensation of a gaseous stream comprising ammonia, carbon dioxide, and water.

The various sections of a urea-producing plant are configured to process fluid compositions comprising liquids and/or gases, at various pressures. These fluid compositions are transferred from one device to another, and it is important to control the flow and pressure of these fluid compositions. A urea-producing plant comprises a plurality of pressure control valves, and it is an advantage that at least one of these pressure control valves is a pressure control valve according to the first aspect. A pressure control valve according to the first aspect has an increased lifetime and/or increased reliability compared to conventional pressure control valve wherein the valve seat is made of a single material.

In some embodiments, the urea-producing plant comprises a hydrolyzer section, also called a wastewater section. The hydrolyzer section may be configured to receive streams comprising urea, in particular a low amount, for example less than 10.0 weight%, of urea, and to decompose urea to ammonia and carbon dioxide. The hydrolyzer section may be configured to produce gaseous streams comprising ammonia and carbon dioxide. The hydrolyzer section may be connected to the condensation section of the urea-producing plant, such that the gaseous streams produced therein may be condensed into liquid streams and recycled back to the synthesis plant.

In another aspect, the present disclosure provides the use of a pressure control valve according to the first aspect in a urea-producing plant.

Figure 1 shows an embodiment of a pressure valve **1** according to the first aspect. The pressure control valve **1** comprises a body comprising a first body part **2** and a second body part **3** fluidly connected to each other through a valve seat **8.** The first body part **2** and the second body part **3** are joined with each other with screws **12** and made of the same first material, such as an austenitic stainless steel or duplex stainless steel or super duplex stainless steel. The first body part **2** comprises a pressure control valve fluid inlet **4** for a fluid, and the second body part **3** comprises a pressure control valve fluid outlet **5** for a fluid. The pressure control valve **1** comprises a flow-regulating component comprising a disc **6** and a stem **7** connected to the disc **6.** The flow regulating component is made of the third material. The stem **7** extends beyond the first body part **2** and can be actuated by an operator or a controller to regulate the position of the disc **6,** and to regulate the volume flow rate of a fluid from the first body part **2** to the second body part **3.** The pressure control valve **1** comprises a valve seat **8**, wherein the valve seat **8** has a cylindrical shape and circular walls , the valve seat **8** is in contact with the first body part **2** and the second body part **3**, and the valve seat **8** is configured to act as a gasket between the first body part **2** and the second body part **3**. The valve seat comprises an inner region **10** and an outer region **9**, wherein the outer region **9** is made of a second material having a hardness different than that of the first material. The inner region **10** comprises a chamber **11** for allowing a fluid to flow from the first body part **2** to the second body part **3**, and the inner region **10** is made of a third material, such as a super duplex stainless steel, which possesses a high resistance to carbamate corrosion and erosion.

## Claims

1. A pressure control valve comprising a first body part and a second body part fluidically connected to each other through a valve seat, wherein:
- the first body part comprises a pressure control valve fluid inlet and a flow-regulating component, the movement of which regulates the volume flow rate of fluid flowing from the first body part to the second body part;
- the second body part comprises a pressure control valve fluid outlet;
- the first body part and the second body part are made from the same first material;
**characterized in that**
the valve seat has a cylindrical shape and circular walls and comprises an inner region and an outer region, wherein the outer region is made from a second material, the inner region is made from a third material, and the second material has a lower hardness than the third material.

2. The pressure control valve according to claim 1, wherein the valve seat is a gasket.

3. The pressure control valve according to claim 1 or 2, wherein the first material and the third material are identical.

4. The pressure control valve according to any one of claims 1 to 3, wherein the first material is an austenitic stainless steel, a duplex stainless steel, or a super duplex stainless steel.

5. The pressure control valve according to any one of claims 1 to 4, wherein the third material is a super duplex stainless steel.

6. The pressure control valve according to any one of claims 1 to 5, wherein the second material is an austenitic stainless steel.

7. The pressure control valve according to any one of claims 1 to 6, wherein the flow-regulating component comprises a stem and a disc.

8. The pressure control valve according to any one of claims 1 to 6, wherein the flow-regulating component comprises a disc selected from the group consisting of a parabolic-type disc, a needle-type disc, a ball-type disc, and a plug-type disc.

9. The pressure control according to any one of claims 1 to 7, wherein the flow-regulating component is made partially or fully of the third material.

10. A urea-producing plant comprising one or more pressure control valves according to any one of claims 1 to 8.

11. The use of a pressure control valve according to any one of claims 1 to 8 in a urea-producing plant.
